Europäisches Patentamt

European Patent Office

Office européen des brevets

(11) Veröffentlichungsnummer: **0 233 551**
**A2**

(19)

(12) # EUROPÄISCHE PATENTANMELDUNG

(21) Anmeldenummer: **87101535.0**

(22) Anmeldetag: **05.02.87**

(51) Int. Cl.⁴: **A61M 16/00 , A61B 5/00 ,**
**A61H 31/00**

(30) Priorität: **17.02.86 DE 3604986**

(43) Veröffentlichungstag der Anmeldung:
**26.08.87 Patentblatt 87/35**

(84) Benannte Vertragsstaaten:
**AT CH FR GB LI SE**

(71) Anmelder: **Hellige GmbH**
**Postfach 728 Heinrich-von-Stephan-Strasse**
**4**
**D-7800 Freiburg im Breisgau(DE)**

(72) Erfinder: **Schläfke, Marianne E., Prof. Dr.**
**Paracelsusweg 20**
**D-4630 Bochum 1(DE)**
Erfinder: **Hopmeier, Joachim**
**Tiroler Weg 12**
**D-7800 Freiburg(DE)**

(74) Vertreter: **Patentanwälte TER MEER - MÜLLER**
**- STEINMEISTER**
**Mauerkircherstrasse 45**
**D-8000 München 80(DE)**

(54) **Einrichtung zur Verhinderung von Sauerstoffmangelschäden.**

(57) Die Erfindung betrifft ein medizinisches Gerät zur Verhinderung von Sauerstoffmangelschäden und zur Behebung von Atmungsstörungen. Von Meßgeräten (2) werden über an der Körperoberfläche des Patienten (5) angebrachte Sensoren (1) Größen ermittelt, die mit der Atmung bzw. mit der Sauerstoffversorgung des Gewebes im Zusammenhang stehen. Sobald mindestens ein Meßwert die einstellbaren Alarmgrenzen über-bzw. unterschreitet, wird das Alarmsignal an eine Alarmverknüpfungseinrichtung (3) weitergeleitet. Von der Alarmverknüpfungseinrichtung (3) werden Schalteinrichtungen (4.1) und (4.2) gesteuert, die einerseits über geeignete Reizeinrichtungen (6.1) bis (6.3) Sinnesreize als unspezifische Atemweckreize (8.1) und andererseits spezifische Atemreize (8.3) durch Anblasen des Patienten (5) mit einer speziellen Atemgasmischung (7.1) abgeben. Sofern die abgegebenen Reize nicht den gewünschten Effekt zeigen, was über die Meßgeräte (2) fortlaufend geprüft wird, kann automatisch die Art, die Dauer, die Folge bzw. die Intensität der Reize so lange geändert werden, wie es den individuellen Bedürfnissen des Patienten (5) entspricht.

FIG.1

## Einrichtung zur Verhinderung von Sauerstoffmangelschäden

Die Erfindung betrifft eine Einrichtung zur Verhinderung von Sauerstoffmangelschäden und zur Behebung von Atmungsstörungen.

Sauerstoffmangel führt beim Gesunden zu einer deutlichen Atemsteigerung. Bei Abfall des Sauerstoffgehalts im Blut unter einen kritischen Wert treten mit anhaltendem Sauerstoffmangel - schwerwiegende Wirkungen auf, beispielsweise Stoffwechselstörungen durch Anfall von Milchsäure, Durchsäuerung des Gewebes, Zellschädigung; besonders gefährdet sind Gehirn und Herz. Gehirnstörungen können neuromuskuläre Koordinationsstörungen, die Abnahme der Urteilskraft, Sehstörungen, Schweißausbrüche, Kollaps und Bewußtlosigkeit, Hirndrucksteigerung, Krampfanfälle und Verlust von Hirngewebe zur Folge haben. Bei den Herzstörungen wäre auf ventrikuläre Extrasystolen, Kammerflimmern, cor pulmonale - (Rechtsherzbelastung) und Veränderung der Lungengefäßwände hinzuweisen.

Sauerstoffmangelzustände sind häufig Folgen von Atemstörungen. Mangelhafter Atemantrieb durch Störung der Regulationssysteme im Zentralnervensystem äußert sich oftmals nur unter Ruhebedingungen. So können bekanntermaßen Atemstörungen bei Frühgeburtlichkeit infolge unreifer Hirnstrukturen sowie frühkindliche Atmungsstörungen Ursache des sogenannten "plötzlichen Säuglingstods" (SIDS = Sudden Infant Death Syndrome) in den ersten Lebensmonaten sein. Gefährlich sind dabei oft nicht erkannte langfristige Sauerstoffmangelzustände im Schlaf mit pathologischem Ausbleiben einer Atemsteigerung, so daß Schäden im zentralen und autonomen Nervensysteme sowie am Herz-Lungen-System entstehen können.

Der Einsatz von Beatmungsgeräten zur vorübergehenden Behandlung von Atmungsstörungen ist bekannt. Bekannt ist auch die Elektrostimulation der Zwerchfellnerven zur künstlichen Auslösung von Atmungsbewegungen. Ebenfalls bekannt ist die medikamentöse Behandlung derartiger Störungen. Im Zusammenhang mit der Therapie von Atmungsstörungen bei Frühgeborenen ist die Sauerstofftherapie, z. B. das Sauerstoffzelt, häufig angewendet worden. Die bekannte Sauerstofftherapie bei Frühgeborenen kann jedoch zur Erblindung und zu Schäden an der Lunge mit Gasaustauschstörungen führen. .

Sofern zentral-atemgestörte Patienten mit Hilfe von Beatmungsgeräten künstlich beatmet werden, wird zwar der dringend lebensnotwendige Gasaustausch aufrechterhalten, doch Therapiemaßnahmen zur Wiederherstellung der Spontanatmung sind unzureichend. Bei der Anwendung von längerdauernder künstlicher Beatmung ist bekannt, daß Patienten die Fähigkeit zur Spontanatmung zumindest vorübergehend verlieren können.

Bei der Elektrostimulation der Zwerchfellnerven wurden irreversible Schädigungen der Nerven und häufig unkoordinierte Atmungsbewegungen beobachtet, so daß zur Vermeidung von Atemwegsblockaden ein Luftröhrenschnitt nötig ist.

Die medikamentöse Therapie bringt häufig keinen oder nur vorübergehenden Erfolg und kann zu Abhängigkeit und zu einschneidenden Störungen des Schlafverhaltens führen.

Die beschriebenen Behandlungsmethoden haben oft den Nachteil, daß der Patient verlernt, selbständig zu atmen, weil die Atemreflexe nicht trainiert werden. Auch die Reifung des Atmungssystems, die bei Frühgeborenen häufig noch nicht genügend vorangeschritten ist, wird nicht gefördert.

In Ermangelung geeigneter Therapieansätze beschränkt man sich bislang auf den Einsatz von Apnoe-Monitoren, vor allem als Heimgeräte für Säuglinge, die vom plötzlichen Säuglingstod - (SIDS) bedroht sind. Sie erzeugen bei Atemunterbrechungen einen sichtbaren oder hörbaren Alarm, der den Schläfer letztlich weckt. Nachteilig ist, daß mit dieser ausschließlichen Rettungswirkung weder der gefährliche chronische Sauerstoffmangel behoben noch die Reifung des Atemreflexes unterstützt wird.

Wegen der aufgezeigten Gefahren und Nebenwirkungen der bisherigen Therapiemöglichkeiten werden in der Regel nur schwere Atemstörungen behandelt. Die nichtbehandelten Atemstörungen können allerdings andererseits zu schweren Erkrankungen führen (z. B. SIDS, s.o.).

Der Erfindung liegt daher die Aufgabe zugrunde, eine Einrichtung zu schaffen, die es ermöglicht, Atmungsstörungen zu erkennen, den Blutsauerstoffgehalt unverzüglich aufzubessern und gleichzeitig längerfristig die Atemreflexe zu trainieren.

Diese Aufgabe wird erfindungsgemäß durch die im Patentanspruch 1 angegebenen Maßnahmen gelöst.

Vorteilhafte Weiterbildungen des Erfindungsgedankens sind in Unteransprüchen gekennzeichnet.

Mit der erfindungsgemäßen Einrichtung ist es möglich, Atmungsstörungen zu erkennen und zudem sowohl eine schnelle Aufbesserung des Blutsauerstoffgehalts zu erreichen als auch die Atemreflexe zu trainieren. Ein langfristiger Therapieerfolg kann beispielsweise durch Bahnung des zentralen kohlendioxidgesteuerten Chemoreflexes erreicht werden. Mit Unterstützung durch die Einrichtung lernt der Organismus, aus dem circulus vitiosus seiner pathologischen Atmung auszubrechen und langfristig therapieunabhängig zu werden.

Die Vorrichtung eignet sich zur Anwendung beim Atemnotsyndrom des Frühgeborenen, im Bereich der Neonatologie zum Training der z. B. durch perinatale Hypoxien bedingten Störungen des Zentralnervensystems mit Ateminsuffizienz, zur Prophylaxe des SIDS bei Abfall des Blutsauerstoffgehalts im Schlaf während des ersten Lebensjahrs, zum Einsatz bei Beatmungspatienten, z. B. in der Entwöhnungsphase, beim Schlaf-Apnoe-Syndrom des Erwachsenen sowie im Bereich der Neurochirurgie bei passageren zentralen Atemstörungen nach Operationen und Traumen.

Die erfindungsgemäße Einrichtung mißt wenigstens eine, mit der Atmung oder der Sauerstoffversorgung des Gewebes verbundene physiologische Größe und veranlaßt bei Über-oder Unterschreitung von Grenzwerten therapeutische Maßnahmen. Überwacht werden beispielsweise die Atemfrequenz, die Atemtiefe, die Herz-oder Pulsfrequenz, der Sauerstoff-oder Kohlendioxidgehalt des Bluts. Die bei Sauerstoffmangel zu beobachtenden Symptome wie Schweißsekretion oder veränderter Hirndruck können weitere wichtige Meßgrößen liefern.

Welche Kombination von Meßgeräten zur Erfassung physiologischer Größen für den jeweiligen Patienten am besten geeignet ist, wird bei einer genauen Voruntersuchung des Patienten festgelegt. Es werden vorzugsweise diejenigen Parameterkombinationen genutzt, die das Krankheitsbild am deutlichsten signalisieren und am sichersten, möglichst auch mit geringer Schlafstörung, meßbar sind. Die Voruntersuchung des Patienten kann ergeben, daß es genügt, die Herzfrequenz auf pathologische Einbrüche, z. B. Bradykardien als Zeichen überlanger Atempausen, hin zu verfolgen. Andere Patienten haben Bradykardien ohne Zusammenhang mit Atempausen. Fehlalarme können dann durch Überwachung zusätzlicher Meßgrößen, z. B. der Atemfrequenz, unterbunden werden. Für jede Größe werden bei Über-oder Unterschreitung individueller Grenzwerte Voralarme erzeugt, die nach außen nicht weiter in Erscheinung treten müssen, deren geeignete logische Verknüpfung· aber ein therapieauslösendes Ereignis darstellt. Aus den von der Atmung beeinflußten physiologischen Größen ergeben sich viele Verknüpfungsmöglichkeiten der individuellen Voralarme zum therapieauslösenden Ereignis. Auswahl der Überwachungsgrößen und Verknüpfungsart ihrer Voralarme werden dem Patienten und seinem Krankheitsbild angepaßt mit dem Ziel hohen Therapieerfolgs und minimaler falsch-positiver wie falsch-negativer therapieauslösender Ereignisse. Bewährt hat sich beispielsweise bei Säuglingen mit ·

Schlaf-Apnoe-Syndrom die simultane Überwachung der Herzfrequenz und des transkutan gemessenen Sauerstoffpartialdrucks im Blut, wenn jeder Voralarm bereits die Therapiemaßnahmen auslöst.

Um sofort eine Aufbesserung des Blutsauerstoffgehalts zu erreichen, wird bei therapieauslösendem Ereignis zunächst das Atemzentrum angeregt oder bei Atemstillstand "geweckt", ohne im allgemeinen den Patienten zu wecken. Für diese "Atemweckaktion" kommen vorzugsweise unspezifische Sinnesreize in Frage, wie Licht-, Schall-, Geruchs-oder in der Haut erfaßbare Reize, womit die bei mechanischer Beatmung entstehende Atemhemmung vermieden wird. Auch können gleichzeitig oder zeitverschoben verschiedenartige Atemweckreize ausgelöst werden. Besonders günstig ist es, das Gesicht des Patienten kurzzeitig mit Atemgas anzublasen, welches vorteilhaft eine erhöhte Sauerstoffkonzen tration enthalten kann. Ein Luftzug, z. B. im Nasen-Mund-Bereich, reizt den Trigeminus, dessen Nervenaktivität die Grundaktivität des Atmungszentrums im Sinne eines verbesserten Atemantriebs beeinflußt. Der von einem Atemweckreiz ausgelöste Atemzug ist effektiver, wenn Atemgas mit erhöhter Sauerstoffkonzentration angeboten wird. Kurzzeitig kann Sauerstoff auch direkt in die Nase eingeleitet werden, wobei trotz ausreichender Oxygenierung Frühgeborene normalerweise keine Sauerstoffschädigung mehr erleiden.

Die Erfindung wird nachfolgend unter Bezug auf die Zeichnungen in beispielhafter Ausführungsform näher erläutert. Es zeigen:

Fig. 1 schematisch den apparativen Aufbau des erfindungsgemäßen Therapiesystems zur Behebung von Atmungsstörungen und zur Aufbesserung des Blutsauerstoffgehalts,

Fig. 2 den zeitlichen Ablauf der unspezifischen Atemweckreizung und der spezifischen Atemreizung, und

Fig. 3 die zeitkorrelierte Wiedergabe von physiologischen Meßgrößen, insbesondere der Atmungsaktivität, bei einem unter Atmungsstörungen leidenden Neugeborenen.

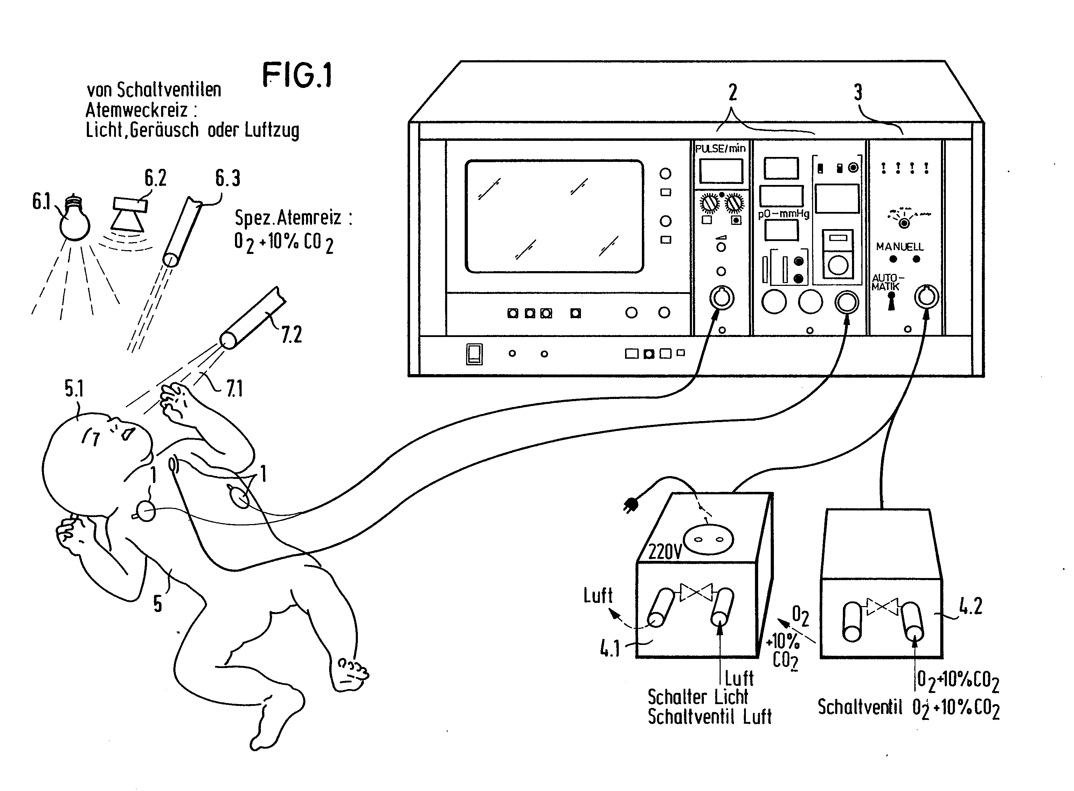

Gemäß Fig. 1 mißt das erfindungsgemäße Gerät über Sensoren 1, die auf der Haut des Patienten 5 angebracht sind, physiologische Größen, die mit der Atmung oder der Sauerstoffversorgung des Gewebes verbunden sind. Überwacht werden durch entsprechende Meßgeräte 2 beispielsweise die Atemfrequenz, die Atemtiefe, die Herzfrequenz, die Pulsfrequenz oder der Sauerstoff-oder Kohlendioxidgehalt des Bluts. Auch die Schweißsekretion oder der Hirndruck können wichtige Meßgrößen sein.

Die Meßgeräte 2 sind mit einstellbaren Alarmeinrichtungen versehen, so daß zunächst intern Alarm ausgelöst wird, sofern der Meßwert den oberen Alarmgrenzwert überschreitet oder den unteren Alarmgrenzwert unterschreitet.

Die von den Meßgeräten 2 festgestellten Alarme sind "Voralarme" und führen nur in bestimmten Sonderfällen zu therapieauslösenden Maßnahmen. Welche Kombination von Meßgeräten 2 für einen bestimmten Patienten am besten geeignet ist, wird, wie bereits erwähnt, bei einer genauen Voruntersuchung des Patienten festgelegt.

Von den Meßgeräten 2 werden die Alarmsignale weitergeleitet an eine Alarmverknüpfungseinrichtung 3, mit deren Hilfe die individuellen Voralarme -wie bereits oben erläutert -zu einem therapieauslösenden Ereignis verknüpft werden. Die Alarmverknüpfungseinrichtung 3 betätigt mit ihren Ausgangssignalen Schalteinrichtungen 4.1 und 4.2.

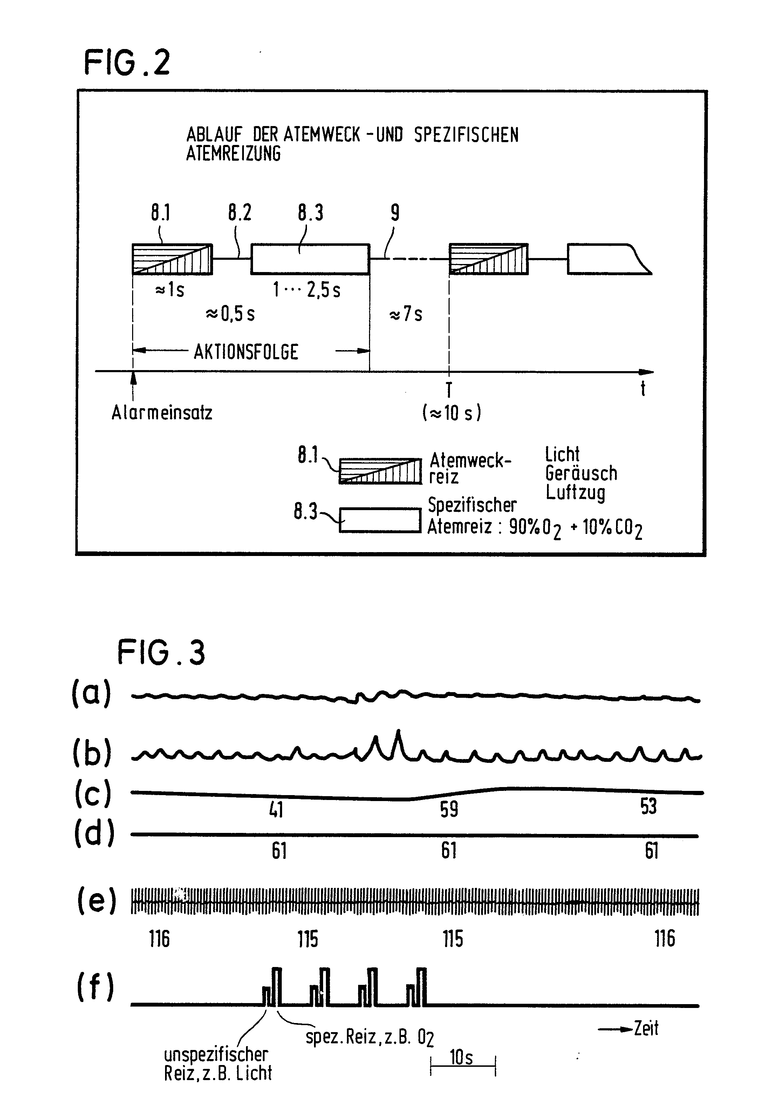

Über die Schalteinrichtung 4.1 wird der Teil einer Aktionsfolge 8 (vgl. Fig. 2) gesteuert, der einem unspezifischen Atemweckreiz 8.1 entspricht. Der unspezifische Atemweckreiz 8.1 kann aus verschiedenen Sinnesreizen bestehen, so beispielsweise aus Licht-, Schall-, Bewegungs-, Geruchsoder durch die Haut empfindbaren Reizen wie Anblasen, Kitzeln oder Wärme-/Kälteeinwirkung.

In der Nähe des Kopfs 5.1 des Patienten 5 sind mehrere Reizeinrichtungen 6.1 bis 6.3 angebracht, über die dem Patienten 5 der unspezifische Atemweckreiz 8.1 zugeführt wird. Als Reizeinrichtungen 6 sind beispielsweise Glühbirnen, Blitzlampen,Lautsprecher, Schläuche mit ausströmender Luft o. ä. geeignet.

Der unspezifische Atemweckreiz 8.1 kann aus einer Kombination mehrerer Sinnesreize bestehen, so beispielsweise aus einem Lichtreiz und einem Schallreiz. Die verschiedenen Sinnesreize können gleichzeitig oder gegeneinander zeitversetzt an den Patienten 5 abgegeben werden. Die Dauer des unspezifischen Atemweckreizes beträgt etwa 0,2 bis 5 Sekunden, vorzugsweise 0,5 bis 2 Sekunden. Über die Schalteinrichtung 4.2 wird dem Patienten ein weiterer Teil der Aktionsfolge 8, nämlich der spezifische Atemreiz 8.3 zugeführt. Während der Dauer des spezifischen Atemreizes 8.3 wird der Patient 5 mit einem speziellen Atemgasgemisch 7.1, das im Vergleich zu normaler Umgebungsluft erhöhte Konzentration (en) von Kohlendioxid und/oder Sauerstoff oder andere geeignete Stoffe enthält, mittels eines Schlauchs 7.2 im Nasen-Mund-Bereich angeblasen. Die Dauer des spezifischen Atemreizes 8.3 beträgt etwa 0,2 bis 5 Sekunden, vorzugsweise 0,5 bis 2,0 Sekunden.

Die Dauer der Pause 8.2 zwischen dem unspezifischen Atemweckreiz 8.1 und dem spezifischen Atemreiz 8.3 innerhalb einer einzigen Aktionsfolge 8 beträgt 0,2 bis 5 Sekunden, vorzugsweise 0,3 bis 0,8 Sekunden.

Bei Atmungsstörungen mit mangelhaftem $CO_2$-Atmungsantrieb ist trotz erhöhter Wasserstoffionenkonzentration keine Atemsteigerung zu beobachten. Der hierfür zuständige zentrale Chemoreflex kann jedoch mit spezifischen Atemreizen, z. B. durch Zumischen von Kohlendioxid zum Atemgas mit vorzugsweise gleichzeitig erhöhter Sauerstoffkonzentration, trainiert werden. Damit wird die Chemoempfindlichkeit im zentralen Nervensystem gebahnt. Besonders geeignet ist es beispielsweise, wenn der Patient ein Gasgemisch mit etwa 2% Kohlendioxid und 40 bis 98% Sauerstoff neben Stickstoff einatmet und wenn kurz vor oder gleichzeitig mit dem Angebot des Atemgasstroms ein unspezifischer Atemweckreiz, wie z. B. ein Lichtblitz, ausgelöst wird, da er den Atemantrieb fördert.

Die Dauer der Pause 9 zwischen zwei aufeinanderfolgenden Aktionsfolgen 8 beträgt 2 bis 30 Sekunden, vorzugsweise 5 bis 15 Sekunden.

Der zeitliche Ablauf einer Aktionsfolge 8, bestehend aus der unspezifischen Atemweckreizung 8.1 und der spezifischen Atemreizung 8.3 einschließlich der Pause 8.2 zwischen beiden Reizen, sowie der Pause 9 zwischen zwei aufeinanderfolgenden Aktionsfolgen 8, ist in Fig. 2 ausführlich dargestellt.

Bei ungenügender Verbesserung der Atmung, insbesondere nicht ausreichender Sauerstoffaufbesserung, wird die Aktionsfolge 8 aus Atemweckreiz 8.1 und gegebenenfalls spezifischem Atemreiz 8.3 wiederholt, jedoch frühestens abgebrochen, wenn der therapieauslösende Alarm aufgehoben ist.

Die Fig. 3 gibt den zeitlichen Verlauf verschiedener gemessener physiologischer Größen wieder. Die beiden oberen Signalverläufe (a) und -(b) veranschaulichen die thorakalen bzw. abdominalen Atembewegungen. Die Signalverläufe (c) und (d) zeigen die Werte von transkutan gemessenem Sauerstoff-bzw. Kohlendioxidpartialdruck (tcp$O_2$ bzw. tcp$CO_2$ ). Die Kurve (e) entspricht dem EKG bzw. dem Verlauf der Herzfrequenz. Das Diagramm (f) gibt die durch das erfindungsgemäße Gerät ausgelöste Aktionsfolge wieder, wobei im zeitlichen Abstand von ca. 10 Sekunden insgesamt vier Aktionsfolgen mit einem vorausgehenden unspezifischen Atemweckreiz und einem nach kurzer Pause folgenden spezifischen Atemreiz ausgelöst werden. Ersichtlicherweise führen die beiden ersten Aktionsfolgen (Stimuli) noch zu keiner erwünschten Reaktion insbesondere bei den unbefriedigenden Atem bewegungen im abdominalen Bereich. Mit der dritten Aktionsfolge jedoch werden die Atmungsaktivitäten stark angeregt, um anschließend in den Normalzustand überzugehen. Gleichzeitig steigt der $tcpO_2$ -Wert auf einen normalen Plateauwert an.

In Weiterbildung der Erfindung ist das Gerät mit einer Einrichtung ausgerüstet, die bei andauerndem mangelhaften Therapieerfolg die Aktionsfolge 8 automatisch so abändert und den individuellen Krankheitsbedingungen des Patienten anpaßt, daß die Atmung verbessert wird. Zur Beurteilung des tatsächlich erreichten oder auch langfristig zu erwartenden Therapieerfolgs im Hinblick auf ausreichenden Blutsauerstoffgehalt und selbständige Atmung können auch zusätzliche physiologische Meßgrößen ausgewertet werden, die nicht notwendigerweise zur Erzeugung des therapieauslösenden Alarms überwacht werden. Beispielsweise kann der aktuelle Blutsauerstoffgehalt eine adäquate Alarmgröße sein, während der z.B. mit zeitlicher Verzögerung gemessene Blutkohlendioxidgehalt prognostisch signifikant für den zu erwartenden Therapieerfolg sein kann.

**Ansprüche**

1. Einrichtung zur Verhinderung von Sauerstoffmangelschäden und zur Behebung von Atmungsstörungen,
**gekennzeichnet durch**
-eine Vorrichtung zur raschen Aufbesserung des Blutsauerstoffgehalts und zur Trainierung des Atemreflexes durch Erzeugung wiederholbarer Aktionsfolgen (8) einstellbarer Dauer, die durch Pausen (9) vorgebbarer Länge getrennt sind, wobei die Aktionsfolgen (8) die Zuführung von Atemgasströmen (7.1) zur Aufbesserung des Sauerstoffgehalts im Blut und/oder unspezifische Atemweckreize (8.1) und/oder spezifische Atemreize (8.3) mit Wirkung auf die atemsteuernden Zentren des Nervensystems umfassen, und durch
-Meßgeräte (2) zur Erfassung physiologischer Größen, die bei Über-oder Unterschreitung vorgebbarer Grenzwerte der gemessenen Größen die Aktionsfolgen (8) automatisch auslösen und nach Rückkehr auf normale Meßwerte wieder unterbrechen.

2. Einrichtung nach Anspruch 1, **dadurch gekennzeichnet,** daß die physiologischen Größen, die bei Über-oder Unterschreitung von Grenzwerten zur Auslösung der Aktionsfolgen (8) führen, eine oder mehrere der Meßgrößen wie Atemfrequenz, Atemzugvolumen, Herz-oder Pulsfrequenz, Sauerstoffsättigung oder Sauerstoff-oder Kohlendioxid-Partialdruck des Bluts und/oder andere mit der Atmung oder der Sauerstoffversorgung des Gewebes in Verbindung stehende Größen sind.

3. Einrichtung nach den Ansprüchen 1 und 2, **dadurch gekennzeichnet,** daß die physiologischen Größen nach Über-oder Unterschreitung von Grenzwerten entweder einzeln oder in vorgebbarer Verknüpfung miteinander zur Auslösung der Aktionsfolgen (8) führen und daß dieselbe oder eine andere Verknüpfung bei Rückkehr auf normale Meßwerte zur Unterbrechung der Aktionsfolgen (8) führt.

4. Einrichung nach Anspruch 1, **dadurch gekennzeichnet,** daß die unspezifischen Atemweckreize (8.1) Sinnesreize sind wie Licht-, Schall-, Bewegungs-, Geruchs-oder durch die Haut empfindbare Reize, wie Anblasen, Kitzeln oder Wärme-/Kälteeinwirkung, und daß die spezifischen Atemreize (8.3) im Vergleich zu normaler Umgebungsluft erhöhte Konzentrationen von $CO_2$ und/oder $O_2$ enthalten oder daß das Gasgemisch zum Anblasen des Patienten andere geeignete Stoffe enthält.

5. Einrichtung nach Anspruch 1, **dadurch gekennzeichnet,** daß das Atemgas (7.1) im Vergleich zu normaler Umgebungsluft eine erhöhte Sauerstoffkonzentration zur Aufbesserung des Sauerstoffgehalts im Blut aufweist.

6. Einrichtung nach Anspruch 1, **dadurch gekennzeichnet,** daß die Aktionsfolge (8) mindestens einen unspezifischen Atemweckreiz (8.1) aufweist.

7. Einrichtung nach den Ansprüchen 1 und 6, **dadurch gekennzeichnet,** daß die Aktionsfolge - (8) nach einem ersten unspezifischen Atemweckreiz (8.1) und einer Pause (8.2) einen spezifischen Atemreiz (8.3) enthält.

8. Einrichtung nach den Ansprüchen 1 und 4 bis 7, **dadurch gekennzeichnet,** daß die Dauer der Einzelaktionen (8.1 und 8.3) und die Dauer der Pausen (8.2) innerhalb einer einzelnen Aktionsfolge (8) 0,2 bis 5 Sekunden, vorzugsweise 0,5 bis 2 Sekunden, betragen.

9. Einrichtung nach den Ansprüchen 1 und 4 bis 8, **dadurch gekennzeichnet,** daß die Pause - (9) zwischen zwei aufeinanderfolgenden Aktionsfolgen (8) 2 bis 30 Sekunden, insbesondere 5 bis 15 Sekunden, beträgt.

10. Einrichtung nach den Ansprüchen 1 und 2, **dadurch gekennzeichnet,** daß die Meßgrößen, die zur Auslösung von Aktionsfolgen (8) führen, auf nichtinvasive Weise gemessen werden, z. B. durch auf der Haut des Patienten angebrachte Sensoren - (1).

11. Einrichtung nach einem der vorstehenden Ansprüche, **dadurch gekennzeichnet,** daß die Aufbesserung des Sauerstoffgehalts im Blut durch fortlaufende Messung kontrolliert wird und daß Mittel vorgesehen sind, die bei ausbleibender hinreichender Sauerstoffaufbesserung eine oder mehrere der folgenden Änderungen der Reize bewirken:
-Umschaltung der Art eines unspezifischen Atemweckreizes (8.1), z. B. Umschaltung von Lichtreiz auf Anblasen,

-Änderung, im allgemeinen Erhöhung, des Sauerstoffgehalts im Atemgas (7.1) eines spezifischen Atemreizes (8.3),

-Änderung, im allgemeinen Erhöhung, des Kohlendioxidgehalts im Atemgas (7.1) eines spezifischen Atemreizes (8.3),

- Änderung der Länge eines spezifischen Atemreizes (8.3), beispielsweise Verlängerung eines spezifischen Atemreizes (8.3) mit erhöhtem Sauerstoffgehalt.

12. Einrichtung nach einem der vorstehenden Ansprüche, **gekennzeichnet durch**

-Mittel zur Erfassung der Häufigkeit der Atemstörungen, die zur Auslösung von Reizen geführt haben, über einen wählbaren gleitenden Überwachungszeitraum, und durch

- Mittel, die bei Überschreitung eines wählbaren Schwellenwerts der Häufigkeit der genannten Atemstörungen eine oder mehrere der folgenden Änderungen der Reize bewirken:

--Änderung, im allgemeinen Erhöhung, des Kohlendioxidgehalts im Atemgas (7.1) eines spezifischen Atemreizes,

--Änderung der Länge von spezifischen Atemreizen (8.3), beispielsweise Verlängerung eines spezifischen Atemreizes (8.3) mit erhöhtem Kohlendioxidgehalt,

--Änderung des Sauerstoffgehalts im Atemgas - (7.1) eines spezifischen Atemreizes (8.3),

--Änderung der Pausendauer (9) zwischen wiederholten Aktionsfolgen (8).

# FIG.1

0 233 551

von Schaltventilen
Atemweckreiz :
Licht, Geräusch oder Luftzug

6.1  6.2  6.3

Spez. Atemreiz :
$O_2 + 10\% CO_2$

7.2

7.1

5.1

1

1

5

2  3

PULSE/min

pO-mmHg

MANUELL

AUTO-MATIK

220V

Luft

$O_2$ +10% $CO_2$

4.1

4.2

Luft
Schalter Licht
Schaltventil Luft

$O_2$+10%$CO_2$
Schaltventil $O_2$+10%$CO_2$

# FIG .2

ABLAUF DER ATEMWECK-UND SPEZIFISCHEN ATEMREIZUNG

≈1s

1···2,5s

≈0,5 s

≈7s

AKTIONSFOLGE

Alarmeinsatz

T
(≈10 s)

t

8.1 — Atemweck-reiz

Licht
Geräusch
Luftzug

8.3 — Spezifischer Atemreiz : 90% $O_2$ + 10% $CO_2$

# FIG .3

(a)

(b)

(c)   41   59   53

(d)   61   61   61

(e)
116   115   115   116

(f)

unspezifischer Reiz, z.B. Licht

spez. Reiz, z.B. $O_2$

10s

Zeit